# EUROPEAN PATENT APPLICATION

(11) **EP 2 335 673 A1**
(43) Date of publication of application: **22.06.2011**
(21) Application number: 09015680.3
(22) Date of filing: 18.12.2009
(51) Int. Cl.: A61K 8/04, A61K 8/34, A61K 8/58, A61K 8/73, A61K 8/81, A61Q 5/06

(54) **Hair styling gel foam**

(71) Applicant: KPSS-Kao Professional Salon Services GmbH, 64297 Darmstadt (DE)
(72) Inventor: Sulzbach, Melina, 61440 Oberursel (DE); Weichaus, Dirk, 64404 Bikenbach (DE); Cajan, Christine, 56130 Bad Ems (DE)
(74) Representative: Grit, Mustafa

(57) **Abstract**

Present invention relates to a hair styling gel foam which boosts exceptionally hair volume. The inventors have surprisingly found out that a composition comprising at least one cationic polymer, at least one silicone oil, at least one thickener and a propellant mixture of alkane and dimethylether in an aqueous or aqueous- alcoholic medium has excellent hair styling and restyling benefits together with excellent volumizing and bodifying effects and also exceptional with the cracking sound heard when foam disappears. The hair feels excellently natural both visually and upon touching and looks excellently shiny.

## Description

Aerosol hair styling compositions have been widely used either as a spray, aerosol or non-aerosol, or as foam. In principal, they comprise hair fixing polymers in an aqueous or aqueous alcoholic medium together with a propellant in case of an aerosol composition. Many fixing polymers have been suggested in the literature either synthetic or natural origin. Natural polymers so far suggested do not satisfy the expectations of consumers in terms of hair feel and hold of hair style for a long time and, therefore, overwhelmingly synthetic polymers are used.

From the consumer needs point of view, a preferred styling composition must have certain performance. It is clear that it must have certain level of hair setting effect and additionally it must not take away hair shine and especially importantly it must allow easy hair styling, diminish flyaways, does not effect negatively hair shine, allow restyling and styled hair must feel as natural as possible. For consumers with fine hair it is also important that hair volume is increased whereas hair still looks natural.

The inventors have surprisingly found out that a composition comprising at least one cationic polymer, at least one silicone oil, at least one thickener and a propellant mixture of alkane and dimethylether in an aqueous or aqueous- alcoholic medium has excellent hair styling and restyling benefits together with excellent volumizing and bodifying effects and also exceptional with the cracking sound heard when foam disappears. The hair feels excellently natural both visually and upon touching and looks excellently shiny.

Thus, the object of the present invention is a hair styling foam composition based on an aqueous or aqueous- alcoholic medium comprising at least one cationic polymer, at least one silicone oil, at least one thickening polymer and a propellant mixture of an alkane and dimethylether.

Another object of the present invention is the use of a composition based on an aqueous or aqueous- alcoholic medium comprising at least one cationic polymer, at least one silicone oil, at least one thickening polymer and a propellant mixture of an alkane and dimethylether for increasing and/or boosting hair volume.

Still another subject of the present invention is a process for increasing and/or boosting volume of human hair wherein a composition based on an aqueous or aqueous- alcoholic medium comprising at least one cationic polymer, at least one silicone oil, at least one thickening polymer and a propellant mixture of an alkane and dimethylether is applied onto wet or dry hair and hair is optionally dried without rinsing off the composition.

Foam composition of the present invention comprises at least one cationic polymer. In principal any cationic polymer is suitable for the purposes of the present invention. Suitable examples are Polyquaternium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46, Polyquaternium 24, Polyquaternium 67, and Polyquaternium 72. However, exceptionally good results are obtained with a cationic polymer formed by polymerization of vinylpyrrolidone, methacrylamide, vinylimidazole and 3-methyl-1-vinylimidazolium methylsulfate. A preferred cationic polymer is a polymer formed by polymerization of above mentioned monomers known with its CTFA name Polyquaternium-68 and commercially available under Luviquat Supreme from BASF.

It should be mentioned that foam compositions can comprise combination of two or more cationic polymers. Concentration of cationic polymer or polymers in total is in the range of 0.1 to 15%, preferably 0.5 to 10%, more preferably 0.5 to 7.5 and most preferably 1 to 7.5% by weight calculated to total composition excluding propellants.

Foam compositions of the present invention comprise at least one silicone oil. Here again any silicone oil either volatile and/or non-volatile is suitable for the compositions of the present invention. Preferred silicone oils are volatile silicone oils known with their lNCl name as dimethicone. Volatile silicone oils such as cyclomethicones are also suitably used. Commercially, they are available from various companies for example Dow Corning with the known DC series, Wacker Chemie and Toray silicones. All commercially available volatile silicones are suitable in the compositions of the present invention. Examples to those are DC 200 series and expecially the ones with lower viscosity such as dimethicone with 0.65 cSt and 1 cSt viscosity at ambient temperature.

Foam composition may certainly comprise two or more silicone oils and especially at least one volatile silicone oil in combination with a non-volatile one. Furthermore, aminated silicones such as amodimethicone and arylated silicones comprising at least one aryl group in its molecule such as phenyl methicone, phenyl trimethicone, diphenyl dimethicone, diphenylsiloxy phenyl trimethicone, tetramethyl tetraphenyl trisiloxane, triphenyl trimethicone, tetramethyl tetraphenyl trisiloxane and trimethyl pentaphenyl trisiloxane can be advantageously comprised in the compositions of the present invention.

Concentration of silicone oil in the compositions is between 1 and 20%, preferably 2.5 and 15%, more preferably 2.5 and 10% and most preferably 2.5 and 7.5% by weight calculated to total composition excluding propellant.

Foam compositions comprise at least one thickening polymer. Any polymer known for thickening aqueous and/or aqueous-alcoholic medium is suitable within the meaning of the present invention. Preferred are non-ionic polymer such as cellulose and its derivatives ethyl cellulose, hydroxyetyhlcellulose, guar gum such as hydroxyl propyl guar gum, and xanthan gum. Preferred are cellulose and guar gum and their derivatives and most preferred is hydroxyethylcellulose.

Concentration of thickening polymer is in the range of 0.05 to 2%, preferably 0.05 to 1.5 and more preferably 0.1 to 1% by weight calculated to total composition excluding propellant.

Foam composition is in aerosol from and comprises propellant mixture of alkane with dimethyl ether. Suitable alkanes including haloalkanes are ethane, propane, n-butane, isobutene, pentane, isopentane, chlorodifluoromethane, 1-chloro-1,1-diflorocarbon, 1,1,1,2-tetrafluoroethane and1,1-difluroethane. Most preferred are propane, n-butane and their mixtures.

Aeorol foam composition comprises alkane or mixture of alkanes to dimethyl ether at a weight ratio of 10:1 1 to 1:1, preferably 8:1 to 2:1, more preferably 7:1 to 2.1 and most preferably 6:1 to 3:1 and in particular 5:1 to 4:1. Concentration of total propellants in the foam composition is in the range of 20 to 75%, preferably 30 to 60 and more preferably 35 to 55% by weight calculated to total composition.

Compositions of the present invention can comprise at least one film forming polymer at a concentration of 0.1 to 25%, preferably 1.5 to 20%, more preferably 2.5 to 15 and most preferably 4 to 15% by weight calculated to total composition excluding propellant.

Within the meaning of the present invention at least one film forming polymer is selected from the anionic, non-ionic, cationic and/or amphoteric or zwitterionic ones.

Suitable non-ionic polymer is first of all vinylpyrrolidon polymers either homopolymers or copolymers with, especially, vinylacetate. Those are known with the trade name "Luviskol" as homopolymers Luviskol K 30, K 60 or K 90 as well copolymers Luviskol VA 55, VA 64, Plus from BASF AG and advantage LS-E from ISP.

Natural non-ionic polymers are as well suitable for the composition of the present invention. Those are such as cellulose, chitosan, guar gum, neutralised shellac and their derivatives.

As amphoteric polymers which can be used alone or in mixture with at least one additional nonionic polymer, reference is here made in particular to copolymers of N-octyl acrylamide, (meth)acrylic acid and tert.-butyl aminoethyl methacrylate of the type "Amphomer®"; copolymers from methacryloyl ethyl betaine and alkyl methacrylates of the type "Yukaformer®", e.g.. the butyl methacrylate copolymer "YukaformerO Am75"; copolymers from monomers containing carboxyl groups and sulfonic groups, e.g.. (meth)acrylic acid and itaconic acid, with monomers such as mono- or dialkyl aminoalkyl (meth)acrylates or mono- or dialkyl aminoalkyl (meth)-acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199.

Suitable anionic polymers alone or in combination with non-ionic polymers are vinyl - alkyl ether, in particular methyl vinyl ether/maleic acid copolymers, obtained by hydrolysis of vinyl ether/maleic anhydride copolymers, distributed under the trade name "Gantrez® AN or ES". These polymers may also be partly esterified, as for example, "Gantrez® ES 225" or "ES 435", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g.. "Flexan®130"; ethyl acrylate/acrylic acid/N-tert.-butyl acrylamide copolymers of the type "Ultrahold®"; vinyl pyrrolidone/vinyl acetate/itaconic acid copolymers, acrylic acid/acrylamide copolymers or the sodium salts thereof of the type "Reten®"; etc.

Further suitable anionic polymers are Acrylate copolymers available under trade name Salcare SC 81, PEG/PPG 25/25 dimethicone / acrylate copolymer available under trade name Luviglex Silk from BASF, Acrylates/t-butylacrylamide copolymer available under trade name Ultrahold Strong, Advantage LC-E which is vinylcaprolactam/PVP/dimethylaminoethylmethacrylate copolymer and VA/crotonates copolymer available under trade name Luviset CA 66.

Foam composition comprises at least one alcohol such as ethanol, isopropanol, propoanol etc. Most preferred is ethanol. Concentration of ethanol is in the range of 1 to 35%, preferably 5 to 30%, more preferably 5 to 25% and most preferably 10 to 25% by weight calculated to total composition excluding propellants.

Polyols are comprise in the foam compositions advantageously. Suitable are panthenol, glycerol, glycol, (poly)ethyleneglycol, (poly)propylenglycol, etc. Conentration of polyol varies in the range of 0.05 to 5%, preferably 0.05 to 2.5% by weight, calculated to total composition.

Compositions of the present invention may comprise one or more surfactant for solubilising of one or more ingredients not soluble in the medium. Suitable ones are of anionic, non-ionic, amphoteric and cationic surfactants or their mixtures.

As a rule any cationic surfactant is suitable for the compositions of the present invention. Preferably at least one cationic surfactant is selected from the compounds with the general formula

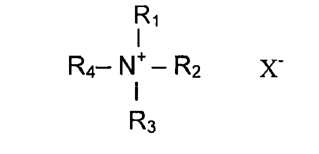

where R₁s a saturated or unsaturated, branched or non-branched alkyl chain with 8-22 C atoms or
R₅CO NH (CH₂)ₙ
where R₅ is saturated or unsaturated, branched or non-branched alkyl chain with 7-21 C atoms and n has typical value of 0 ― 4 or
R₆CO O (CH₂)ₙ
where R₆ is saturated or unsaturated, branched or non-branched alkyl chain with 7-21 C atoms and n has typical value of 0 ― 4, and
and R₂, R₃ and R₄ are independent from each other H or lower alkyl chain with 1 to 4 carbon atoms or ethoxy or propoxy group with number of ethoxy or propoxy groups varying in the range of 0 to 4, and X is chloride, bromide or methosulfate.

Non-limiting examples to suitable cationic surfactants are cetyl trimethyl ammonium chloride, myristoyl trimethyl ammonium chloride, behentrimonium chloride, trimethyl cetyl ammonium bromide, stearyl trimethyl ammonium chloride, dimethyl stearyl ammonium chloride, stearamidopropyldimethyl ammonium chloride.

Suitable non-ionic surfactants are alkyl polyglucosides of the general formula
R₇ ― O ―(R₈O)ₙ O ― Zx
wherein R₇ is an alkyl group with 8 to 18 carbon atoms, R₈ is an ethylene or propylene group, Z is a saccharide group with 5 to 6 carbon atoms, n is a number from 0 to 10 and x is a number between 1 and 5. Examples are decyl polyglucoside, cocoyl polyglucoside both are commercially available.

Further nonionic surfactant components are, for example, long-chain fatty acid mono-and dialkanolamides, such as coco fatty acid mono and di ethanolamide and myristic fatty acid mono and di ethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants useful in the compositions according to invention are C₁₀-C₂₂-fatty alcohol ethoxylates. Suitable non-limiting examples are oleth-10, oleth-11, oleth-12, oleth-15, oleth-16, oleth-20, oleth-25, oleth-30, oleth-35, oleth-40, laureth-10, laureth-11, laureth-12, laureth-13, laureth-15, laureth-16, laureth-20, laureth-25, laureth-30, laureth-35, laureth-40, laureth-50, ceteth-10, ceteth-12, ceteth-14, ceteth-15, ceteth-16, ceteth-17, ceteth-20, ceteth-25, ceteth-30, ceteth-40, ceteth-45, cetoleth-10, cetoleth-12, cetoleth-14, cetoleth-15, cetoleth-16, cetoleth-17, cetoleth-20, cetoleth-25, cetoleth-30, cetoleth-40, cetoleth-45, ceteareth-10, ceteareth-12, ceteareth-14, ceteareth-15, ceteareth-16, ceteareth-18, ceteareth-20, ceteareth-22, ceteareth-25, ceteareth-30, ceteareth-40, ceteareth-45, ceteareth-50, isosteareth-10, isosteareth-12, isosteareth-15, isosteareth-20, isosteareth-22, isosteareth-25, isosteareth-50, steareth-10, steareth-11, steareth-14, steareth-15, steareth-16, steareth-20, steareth-25, steareth-30, steareth-40, steareth-50, steareth-80 and steareth-100. Additional examples of similar compounds can be found in the cosmetic ingredient dictionaries and cosmetic textbooks.

Further non-ionic surfactants within the meaning of the present invention are polyalkyleneglycol ether of fatty acid glyceride or partial glyceride with at least 30 polyalkylene units are with 30 to 1000, preferably 30 to 500, more preferably 30 to 200 and most preferably 40 to 100 polyethyleneglycol units. Examples to those are PEG-30 hydrogenated castor oil, PEG-35 hydrogenated castor oil, PEG-40 hydrogenated castor oil, PEG-45 hydrogenated castor oil, PEG-50 hydrogenated castor oil, PEG-55 hydrogenated castor oil, PEG-60 hydrogenated castor oil, PEG-65 hydrogenated castor oil, PEG-80 hydrogenated castor oil, PEG-100 hydrogenated castor oil, PEG-200 hydrogenated castor oil, PEG-35 castor oil, PEG-50 castor oil, PEG-55 castor oil, PEG-60 castor oil, PEG-80 castor oil, PEG-100 castor oil, PEG-200 castor oil. Additional examples of similar compounds can be found in the cosmetic ingredient dictionaries and cosmetic textbooks.

Further suitable non-ionic surfactants are monoglycerides such as glyceryl stearate, glyceryl palmitate, glyceryl myristate, glyceryl behenate.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate.

Further, suitable within the meaning of the present invention are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono-, di- or tri alkyl phosphates.

Additional anionic surfactants useful are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof of the formula
R₉- (C₂H₄O)ₙ― O - CH₂COOX,
wherein R₉ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

Concentration of one or more surfactant in the compositions according to present invention is in the range of 0.01 to 5%, preferably 0.05 to 2.5% and more preferably 0.1 to 1 % by weight calculated to total composition, excluding propellant. Preferred surfactants are non-ionic, amphoteric and cationic ones. The most preferred is non-ionic surfactants.

Natural oils may be comprised in the foam compositions which are in principal any triglyceride suitable for cosmetic use. Non-limiting examples are avocado oil, coconut oil, palm oil, sesame oil, peanut oil, whale oil, sunflower oil, almond oil, peach kernel oil, wheat germ oil, macadamia nut oil, night primrose oil, jojoba oil, castor oil, or also olive oil, soya oil, and the derivatives thereof. Mineral oils such as paraffin oil and petrolatum may suitably be contained within the scope of the present invention, It should as well be noted that compositions of the present invention can contain mixture of one or more natural oils and mineral oil.

Further, synthetic oil may be comprised and suitable are in particular fatty alcohol fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethylene glycol and polyglyceryl fatty acid esters, cetyl - palmitate, etc.

Concentration of oil either natural or synthetic may be in the range of 0.01 to 5%, preferably 0.05 to 2.5% by weight calculated to total composition, excluding propellant.

The compositions according to the invention may also comprise further agents, such as proteins, for example bamboo protein, and protein hydrolyzates and polypeptides, e.g. keratin hydrolyzates, collagen hydrolyzates of the type "Nutrilan®" or elastin hydrolyzates, as well as, in particular vegetable, optionally cationized protein hydrolyzates, for example "Gluadin®".

Additional natural plant extracts can as well form part of the compositions of the present invention. Those are incorporated usually in an amount of about 0.01 % to about 5 %, preferably 0.05 % to 3.5 %, in particular 0.1 % to 2 % by weight, calculated as dry residue thereof to the total composition . Suitable aqueous (e.g. steam-distilled) alcoholic or hydro-alcoholic plant extracts known per se are in particular extracts from leaves, fruits, blossoms, roots, rinds or stems of aloe, pineapple, artichoke, arnica, avocado, valerian, bamboo, henbane, birch, stinging nettle, echinacea, ivy, wild angelica, gentian, ferns, pine needles, silver weed, ginseng, broom, oat, rose hip, hamamelis, hay flowers, elderberry, hop, coltsfoot, currants, chamomile, carrots, chestnuts, clover, burr root, coconut, cornflower, lime blossom, lily of the valley, marine algae, balm, mistletoe, passion flower, ratanhia, marigold, rosemary, horse chestnut, pink hawthorn, sage, horsetail, yarrow, primrose, nettle, thyme, walnut, wine leaves, white hawthorn, etc.

Suitable trade products are, for example, the various "Extrapon®" products, "Herbasol^{R} ", "Sedaplant^{R}" and "Hexaplant^{R}". Extracts and the preparation thereof are also described in "Hagers Handbuch der pharmazeutischen Praxis". 4^{th} Ed..

Foam compositions of the present invention may contain UV filters either for stabilization of the product colour and/or for protection of hair from environmental influences such as loss of elasticity, loss of hair colour (bleaching effect of sun light). Suitable UV-absorbing substance are Polysilicone-15, 4-Aminobenzoic acid and the esters and salts thereof, 2-phenyl benzimidazole-5-sulfonic acid and the alkali and amine salts thereof, 4-dimethyl aminobenzoic acid and the esters and salts thereof, cinnamic acid and the esters and salts thereof, 4-methoxycinnamic acid and the esters and salts thereof, salicylic acid and the esters and salts thereof, 2,4-dihydroxybenzophenone, 2,2',4,4'-tetrahydroxy- benzophenone, 2-hydroxy-4-methoxybenzophenone and its 5-sulfonic acid or the sodium salt thereof, 2,2'-dihydroxy-4,4'-dimethoxybenzophenone, 2-hydroxy-5-chlorobenzophenone, 2,2'-dihydroxy-4-methoxybenzophenone, 2,2'-dihydroxy-4,4'-dimethoxy-5,5'-disulfobenzo-phenone or the sodium salt thereof, 2-hydroxy-4-octyloxybenzophenone, 2-hydroxy-4-methoxy-4'-methylbenzophenone, 3-benzyl-idenecampher, 3-(4'-sulfo)-benzyl-idenebornane-2-one and the salts thereof and/or 3-(4'-methyl benzylidene)-DL-campher, and/or polysiliocne-15.

The suitable amount of the UV-absorber ranges from about 0.01 % to 1% by weight, calculated to the total composition . Attention should be paid to the stability and solubility especially when using UV filter as salts, e.g. anionic UV filter salts.

In a further embodiment of the present invention, the compositions comprise at least one direct dye for colouring hair. Suitable direct dyes are cationic, anionic, neutral dyes and their mixtures as available commercially from various suppliers and used mainly in semipermanent hair coloration.

Non-limiting examples to cationic dyes are Basic Blue 6, Basic Blue 7, Basic Blue 9, Basic Blue 26, Basic Blue 41, Basic Blue 99, Basic Brown 4, Basic Brown 16, Basic Brown 17, Basic Orange 31, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14, Basic Yellow 57 and Basic Yellow 87.

Further suitable direct dyes are anionic dye. Suitable non-limiting examples are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Further suitable dyes for colouring hair within the meaning of the present invention are those of neutral nitro dyes. Suitable non-limiting examples are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No. 15, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs may also be used as hair colorant within the meaning of the present invention for example henna (red or black), alkanna root, laccaic acid, indigo, logwood powder, madder root and rhubarb powder, etc.

It should be noted that the above dyestuffs are also suitable for use in mixture. When using direct dyes of various categories, their compatibility must be checked.

The above mentioned dyestuffs are also used especially the anionic ones for product colouring at reduced concentrations.

Concentration of direct dyes in the compositions of the present invention is within the range of 0.001 to 5%, preferably 0.01 to 3% and more preferably 0.05 to 2%, and most preferably 0.1 to 1% by weight calculated to total composition, calculated to total composition.

Composition of the present invention may comprise at least one dipeptide. The dipeptide compounds according to the present invention comprise two amino acid moieties. In principal, any dipeptide available either natural or synthetic is suitable for the purposes of the present invention. The synthetic ones are preferred. In one of the preferred embodiment of the present invention the amino acid moeities of dipeptide are selected from arginine, tyrosine, valine, tryptophan, alanine, cysteine, glycine, lysine, proline, hydroxyproline and histidine. The dipetides according to the present invention may certainly be of two different amino acids but at the same time two of the same amino acids.

Non-limiting examples to the suitable dipeptides are the ones commercially available and known with their lNCl name as Dipeptide-1, Dipeptide-2, Dipeptide-3, Dipeptide-4, Dipeptide-5, Dipeptide-6, Dipeptide-7, Dipeptide-8, and carnosine. The most preferred is carnosine and is of ß-alanin and L-histidine.

Concentration of at least one dipeptide is in the range of 0.01 to 5%, preferably 0.05 to 3% and more preferably 0.1 to 2.5% and most preferably 0.2 to 1.5% by weight calculated to the total composition, excluding propellant.

Furthermore compositions of the present invention can comprise all substances customarily found in such preparations. Examples of such substances are complexing agents, preservatives, fragrances, etc.

The following examples are to illustrate the invention but not to limit.

**Example 1**

| | % by weight |
|---|---|
| Trisiloxane 1 cSt | 7.0 |
| Polyquaternium-68 | 3.5 |
| Glycerin | 1.0 |
| Hydroxyethylcellulose | 0.5 |
| Benzophenone-3 | 0.05 |
| Phenyltrimethicone | 0.1 |
| Fragrance | q.s. |
| Ethanol | 20.0 |
| Water | q.s to 100 % |

The above composition was prepared by dissolving ― dispersing all ingredients one by one in water-ethanol mixture

The above composition was filled into an aerosol can with 45% by weight bulk, 45% by weight propane/butane and 10% by weight dimethylether, all values are calculated to total composition.

The hair styled with the above composition is excellently styled and has excellent natural touch and has not lost its shine, and excellent volume giving effect was observed.

Additionally, cracking sound was heard when foam was taken out and braking.

Similar results were observed with the compositions below.

**Example 2**

| | % by weight |
|---|---|
| Trisiloxane 1 cSt | 3.0 |
| Isopropylpalmitate | 0.1 |
| Polyquaternium-68 | 4.0 |
| Panthenol | 0.05 |
| Hydroxyethylcellulose | 0.6 |
| PVP (Luviskol K 30) | 0.25 |
| Benzophenone-4 | 0.05 |
| Cyclomethicone | 0.25 |
| Fragrance | q.s. |
| Ethanol | 20.0 |
| Water | q.s to 100 % |

The above composition was filled into an aerosol can with 50% by weight bulk, 40% by weight propane/butane and 10% by weight dimethylether, all values are calculated to total composition.

## Claims

1. Hair styling foam composition based on an aqueous or aqueous- alcoholic medium **characterized in that** it comprises at least one cationic polymer, at least one silicone oil, at least one thickening polymer and a propellant mixture of an alkane and dimethylether.

2. Composition according to claim 1 **characterized in that** cationic polymer is selected from the ones formed by polymerization of vinylpyrrolidone, methacrylamide, vinylimidazole and 3-methyl-1-vinylimidazolium methylsulfate.

3. Composition according to claim 1 **characterized in that** cationic polymer is Polyquaternium-68.

4. Composition according to any of the preceding claims **characterized in that** it comprises at least one volatile silicone oil, preferably with a viscosity of 0.65 and 1 cSt at ambient temperature.

5. Composition according to any of the preceding claims **characterized in that** thickening polymer is a non-ionic polymer, and preferably selected from cellulose and its derivatives and guar gum and its derivatives.

6. Composition according to any of the preceding claims **characterized in that** at least one alkane is selected from ethane, propane, n-butane, isobutene, pentane, isopentane, chlorodifluoromethane, 1-chloro-1,1-diflorocarbon, 1,1,1,2-tetrafluoroethane and1,1-difluroethane.

7. Composition according to any of the preceding claims **characterized in that** alkane and dimethylether are comprised at a concentration of 20 and 75% by weight calculated to total composition and at a alkane to dimethylether weight ratio of 10:1 to 1:1.

8. Composition according to any of the preceding claims **characterised in that** it comprises at least one film forming polymer.

9. Composition according to any of the preceding claims **characterized in that** it comprises at least one surfactant, preferably a non-ionic surfactant.

10. Composition according to any of the preceding claims **characterized in that** it comprises at least one polyol.

11. Composition according to any of the preceding claims **characterized in that** it comprises natural and/or synthetic oil.

12. Composition according to any of the preceding claims **characterized in that** it comprises at least one direct dye.

13. Composition according to any of the preceding claims **characterized in that** it comprises at least one UV filter.

14. Use of an aqueous composition according to claims 1 to 13 for increasing and/or boosting hair volume.

15. Process for increasing and/or boosting hair volume wherein a composition according to claims 1 to 13 is applied onto wet and/or dry hair and hair is optionally dried without rinsing off.
